# EUROPEAN PATENT APPLICATION

(11) **EP 3 342 437 A1**
(43) Date of publication of application: **04.07.2018**
(21) Application number: 17150005.1
(22) Date of filing: 02.01.2017
(51) Int. Cl.: A61M 5/14

(54) **PREFILLED INFUSION SET**

(71) Applicant: Fresenius Kabi Deutschland GmbH, 61352 Bad Homburg (DE)
(72) Inventor: PÜTTER, Harry, 36364 Bad Salzschlirf (DE)
(74) Representative: Kusche, Robert

(57) **Abstract**

An infusion set (1) for administering a medical fluid to a patient (P) comprises an inlet, an outlet, a flexible tube (12) providing for a fluid path in between the inlet and the outlet, and a drip chamber (11) arranged on the flexible tube (12) at a location in between the inlet and the outlet. Herein, the infusion set (1), in a delivery state, is closed at the inlet and the outlet in a fluid-tight manner, wherein at least the tube (12) is prefilled with a medical solution in the delivery state. In this way an infusion set is provided which allows for a more efficient workflow in clinical practice for administering a medical solution to a patient.

## Description

The invention relates to an infusion set according to the preamble of claim 1.

An infusion set of this kind serves to administer a medical fluid to a patient. The infusion set may for example be connected at one end to a container such as a flexible bag containing a medical fluid and at another end to a patient such that the medical fluid may flow from the container through the infusion set towards the patient. In this way, a medical solution such as a medicational solution, a nutritional solution for the enteral or parenteral feeding of the patient or another medical solution such as a saline solution may be administered to the patient.

An infusion set of this kind typically comprises an inlet, an outlet, a flexible tube providing for a fluid path in between the inlet and the outlet, and a drip chamber arranged on the flexible tube at a location in between the inlet and the outlet.

Nowadays, the preparation of an infusion set for administering a medical solution to a patient is time-consuming. In particular, during preparation the infusion set must be filled with a medical solution in order to remove air from the infusion set prior to the start of the actual infusion. For this, the infusion set must be connected to a container such as a flexible bag or a glass bottle containing the medical solution to be administered to the patient, upon which it must be waited until the solution has flown into the infusion set, generally driven by gravity. Overall, multiple steps for the preparation are necessary, including the removing of the infusion set from a packaging, the closing off a roller clamp of the infusion set, the connecting of the infusion set to the container, the setting of a liquid level in the drip chamber, the opening of the roller clamp in order to allow the solution to flow into the tube of the infusion set (driven by gravity), the closing of the roller clamp when the tube is filled, and the connection of the infusion set to the patient. Altogether, the preparational steps for filling the infusion set prior to the start of the actual infusion may take up to several minutes, hence placing a significant burden on a user, for example a nurse, in clinical practice.

It is an object of the invention to provide an infusion set which allows for a more efficient workflow in clinical practice for administering a medical solution to a patient.

Accordingly, the infusion set, in a delivery state, is closed at the inlet and the outlet in a fluid-tight manner, wherein at least the tube is prefilled with a medical solution in the delivery state.

The invention allows to deliver an infusion set in a prefilled manner. Hence, the infusion set may be prefilled from a manufacturer and may be delivered to a place of use, for example a hospital, in a prefilled manner such that the preparation of an infusion at the place of use, in particular a hospital, becomes significantly easier.

In the context of this text the delivery state is to be understood as a state in which the infusion set may be delivered from for example a manufacturer to a place of use. Hence, the delivery state corresponds to a state prior to the actual use of the infusion set, hence a state in which the infusion set may be transported and/or stored for a later use.

Because the infusion set is prefilled, any such steps which are nowadays required to fill the infusion set during preparation immediately prior to an infusion process to be able to carry out the actual infusion operation are dispensable. The prefilled infusion set, after removing it from a packaging, may be directly connected to a container at its inlet and to the patient at its outlet, and infusion may immediately start. No extra preparation steps for the filling of the infusion set are required any longer.

The tube of the infusion set may be prefilled with a saline solution. Hence, the infusion set may be delivered (transported and/or stored) in a state in which it is prefilled with a saline solution.

Because the infusion set may be stored for a significant amount of time (possibly multiple years) before it actually is used for an infusion operation, the infusion set should maintain its filling state and its filling volume over a prolonged time. For this, the tube may for example comprise a tube wall being substantially impermeable for the medical solution it is filled with.

To obtain said minimum permeability the tube may be made from a material offering such permeability. Alternatively or in addition, the tube wall may comprise a coating further reducing the permeability of the tube.

The tube may for example be fabricated by co-extrusion.

To close the infusion set at its inlet and its outlet, a fluid-tight end cap may be placed at the inlet and/or the outlet. By means of the fluid-tight end cap it may be ensured that medical solution may not exit from the infusion set at the inlet or the outlet. Furthermore, the fluid-tight end cap may be air-tight such that air may not enter into the infusion set.

The drip chamber may for example be placed at the inlet of the infusion set. The tube in this case is connected to an outlet opening of the drip chamber and extends from the drip chamber towards the outlet of the infusion set. For example, the drip chamber may comprise a connector constituting the inlet of the infusion set, the connector for example being formed as a spike which can be used to pierce a membrane at a port of a container such as a flexible bag or a glass bottle containing a medical solution to be administered to a patient.

In one embodiment, the connector comprises an inlet opening to allow a solution to flow into the drip chamber and a vent opening to allow air to exit from the drip chamber, the inlet opening and the vent opening being closed by an end cap in a fluid-tight manner in the delivery state. An end cap at the inlet of the infusion set is hence used to close off both an inlet opening of the connector for letting the medical solution into the infusion set and a vent opening to allow air to exit from the drip chamber. Hence, by means of the end cap it is prevented that the medical solution may exit from the prefilled infusion set through the inlet opening of the connector or through the vent opening.

In one embodiment, the drip comprises an inner volume prefilled with a predefined volume of the medical solution in the delivery state. Hence, not only the tube of the infusion set is prefilled, but also the drip chamber, wherein the filling level of the drip chamber corresponds to a predefined volume which is chosen such that infusion may immediately start without any further preparational steps for adapting the filling level of the infusion set.

In one embodiment, the drip chamber comprises an outlet opening being in fluid connection with the tube, wherein the drip chamber comprises a hydrophilic membrane separating the inner volume of the drip chamber from the outlet opening. By means of the hydrophilic membrane it can be prevented that air from the inner volume of the drip chamber may enter into the tube during transport or storage of the infusion set, such that no air bubbles may arise in the tube connected to the drip chamber.

In one embodiment, the infusion set is enclosed in a covering container, for example a flexible packaging, to form a delivery arrangement. Beneficially, the covering container is formed by one or multiple barrier sheets, the sheets having a minimum permeability for the medical solution. Hence, the covering container provides for an additional barrier to avoid a diffusion of the medical solution from the infusion set towards the outside during a prolonged storage period. In this way, it is ensured that the filling volume of the infusion set, in particular the filling volume of the medical solution in the tube and in the drip chamber, remains approximately constant over a prolonged storage period, and also the concentration of the medical solution, for example the concentration of a saline solution, remains approximately constant.

The idea underlying the invention shall subsequently be described in more detail with reference to the embodiments shown in the drawings. Herein:
- Fig. 1: shows a schematic drawing of an infusion set;
- Fig. 2: shows an embodiment of an infusion set in a delivery state in which it is prefilled with a medical solution;
- Fig. 3: shows a view of the infusion set packaged within a covering container; and
- Fig. 4: shows a schematic view of a drip chamber of the infusion set.

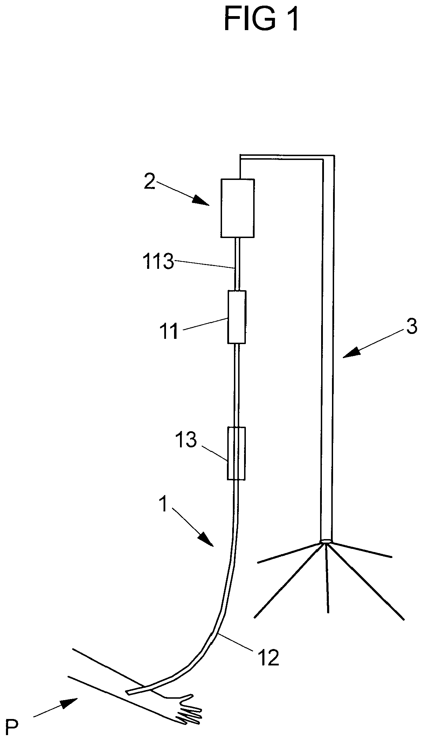

Fig. 1 shows a schematic drawing of an infusion set 1 comprising a drip chamber 11 being connected, via a connector 113 in the shape of a spike, to a container 2, for example a flexible bag, containing a medical solution and being arranged on a stand 3. A tube 12 is connected to the drip chamber 11 such that the medical solution may flow from the container 2 via the drip chamber 11 into the tube 12 and via the tube 12 towards a patient P. A roller clamp 13 is arranged on the tube 12 such that the infusion rate may be adjusted when the medical solution flows through the tube 12 driven by gravity.

During an infusion operation, it must be made sure that no air enters into the patient P. Hence, it must be made sure that the infusion set 1, in particular the tube 12 of the infusion set 1, is filled with medical solution prior to beginning the actual infusion operation.

Conventionally, the removal of air from the infusion set 1 during preparation of an infusion is time-consuming. In particular, the removal of air from the infusion set 1 takes place by filling the infusion set 1, in particular the tube 12, with medical solution, which requires
- the removal of the infusion set 1 from a packaging,
- the closing of the roller clamp 13,
- the connection of the drip chamber 11 to the container 2,
- the opening of the roller camp 13 in order to allow medical solution to flow into the tube 12 and
- again the closing of the roller clamp 13 once the tube 12 is filled with medical solution.
Because the filling of the tube 12 is typically driven by gravity, the filling process (to be carried out by a user, for example a nurse) conventionally may take up to several minutes.

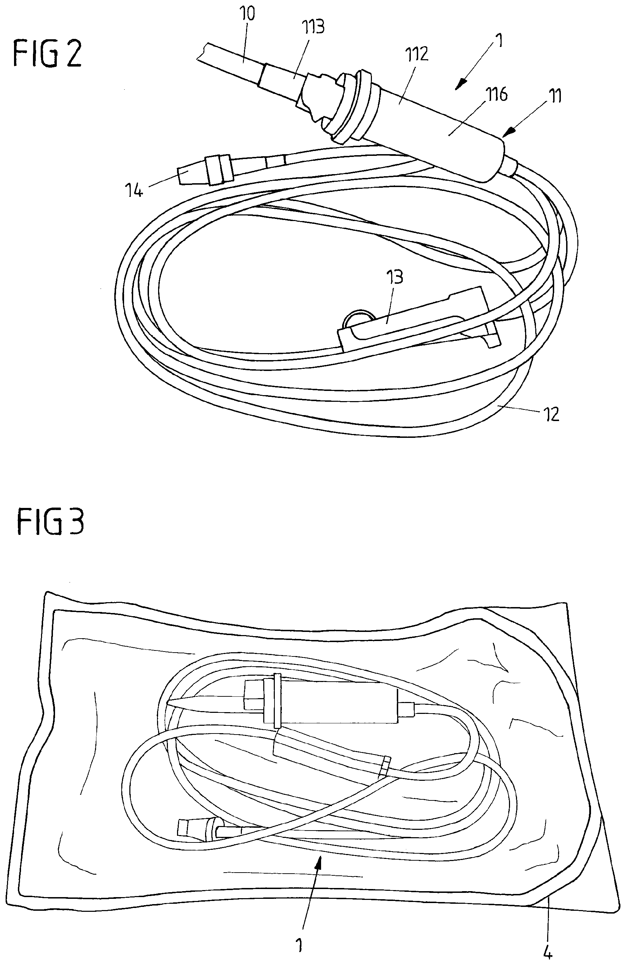

To ease the work of a user, in particular a nurse, it herein is proposed to deliver an infusion set 1 in a prefilled manner. Hence, in a delivery state shown in Fig. 2 the infusion set 1 already is filled with a medical solution, for example a saline solution, such that no air is present in the tube 12, and the drip chamber 11 is already filled with a desired filling volume to be able to readily start an infusion process without the requirement of any additional preparational filling steps.

To allow for a delivering of the infusion set 1 in a prefilled manner, the infusion set 1 must be sealed in a fluid-tight manner such that it maintains its filling level after prefilling and during storage over a prolonged time period. For this, the infusion set 1 is closed off at its inlet (constituted by the connector 113 of the drip chamber 11) by a first end cap 10 and at its outlet, constituted by the far end of the tube 12, by a second end cap 14. The end caps 10, 14 provide for a fluid-tight closing such that no medical solution may exit from the infusion set 1 at the inlet or the outlet and no air may enter into the infusion set 1.

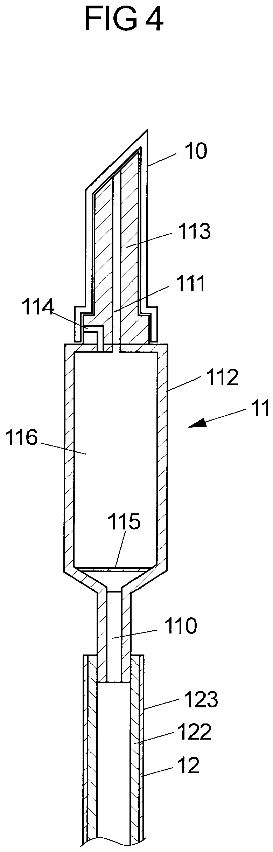

The drip chamber 11, as it is schematically shown in Fig. 4, comprises a body 112 enclosing an inner volume 116, an inlet opening 111 through which medical solution is to flow into the inner volume 116 of the drip chamber 11 once the drip chamber 11 is connected to a container 2 comprising a medical solution. The inlet opening 111 extends through the connector 113 constituted as a spike such that the inlet opening 111 forms a channel through the connector 113 through which medical solution may enter into the inner volume 116 of the drip chamber 11.

As schematically illustrated in Fig. 4, the connector 113 also comprises a vent opening 114 allowing air to exit from the inner volume 116 during an infusion process. Herein, the end cap 10 (schematically shown in Fig. 4) closes off both the inlet opening 111 and the vent opening 114 in the delivery state, such that medical solution may not exit from the inner volume 116 through the inlet opening 111 or through the vent opening 114 when closed off by the end cap 10.

The drip chamber 11, at its opposite end, comprises an outlet opening 110 which is in fluid connection with the tube 12. Herein, a hydrophilic membrane 115 separates the inner volume 116 from the outlet opening 110, such that air may not enter from the inner volume 116 through the membrane 115 into the tube 12 in the prefilled delivery state of the infusion set 1.

The tube 12 is substantially impermeable for the medical solution (i.e., it comprises a minimum permeability) such that medical solution may not diffuse out of the tube 12 over a prolonged duration of storage (for example multiple years). In this way it is made sure that the filling volume of the infusion set 1 remains approximately constant over a prolonged storage period, hence the concentration of for example a saline solution filling the infusion set 1 remaining constant over the prolonged storage period.

To obtain a desired minimum permeability, the tube 12 may be made from a substantially impermeable material. For example, the tube 12 may be fabricated by co-extrusion.

In order to furthermore reduce the permeability, the tube 12 may comprise an additional coating 123 at the outside or at the inside of a tube wall 122 forming the tube 12.

As an additional measure to reduce diffusion of medical solution out of the infusion set 1, the infusion set 1 may be included in a covering container 4 as shown in Fig. 3, the covering container 4 being formed for example from flexible sheets enclosing the infusion set 1 in a tightly-sealed, sterile manner. The covering container 4 may be fabricated from a material having a low permeability for the medical solution.

Additional measures may be to store the infusion set 1 under pressure within the covering container 4.

The invention is not limited to the embodiments described above, but may be implemented in an entirely different fashion in entirely different embodiments.

An infusion set may also be used on an infusion device such as an infusion pump.

The infusion set may comprise multiple tubes for example to be connected to multiple containers. The invention hence is not limited to embodiments comprising only a single tube.

### List of Reference Numerals

- 1: Infusion set
- 10: End cap
- 11: Drip chamber
- 110: Outlet opening
- 111: Inlet opening
- 112: Body
- 113: Connector (spike)
- 114: Vent opening
- 115: Membrane
- 116: Inner volume
- 12: Tube
- 120, 121: End
- 122: Tube wall
- 123: Coating
- 13: Roller clamp
- 14: End cap
- 2: Container (Flexible bag)
- 3: Stand
- P: Patient

## Claims

1. Infusion set (1) for administering a medical fluid to a patient (P), comprising:
- an inlet,
- an outlet,
- a flexible tube (12) providing for a fluid path in between the inlet and the outlet, and
- a drip chamber (11) arranged on the flexible tube (12) at a location in between the inlet and the outlet,
**characterized in**
**that** the infusion set (1), in a delivery state, is closed at the inlet and the outlet in a fluid-tight manner, wherein at least the tube (12) is prefilled with a medical solution in the delivery state.

2. Infusion set (1) according to claim 1, **characterized in that** the tube (12) is prefilled with a saline solution.

3. Infusion set (1) according to claim 1 or 2, **characterized in that** the tube (12) comprises a tube wall (122) being substantially impermeable for the medical solution.

4. Infusion set (1) according to claim 3, **characterized in that** the tube wall (122) of the tube (12) comprises a coating (123) to obtain said impermeability.

5. Infusion set (1) according to one of the preceding claims, **characterized in that** the tube (12) is formed by co-extrusion.

6. Infusion set (1) according to one of the preceding claims, **characterized in that** at least one of the inlet and the outlet is closed by a fluid-tight end cap (10, 14).

7. Infusion set (1) according to one of the preceding claims, **characterized in that** the drip chamber (11) comprises a connector (113) constituting the inlet of the infusion set (1), wherein the connector (113) comprises an inlet opening (111) to allow a solution to flow into the drip chamber (11) and a vent opening (114) to allow air to exit from the drip chamber (11), the inlet opening (111) and the vent opening (114) being closed by an end cap (10) in a fluid-tight manner in the delivery state.

8. Infusion set (1) according to one of the preceding claims, **characterized in that** the drip chamber (11) comprises an inner volume (116) prefilled with a predefined volume of the medical solution in the delivery state.

9. Infusion set (1) according to claim 8, **characterized in that** the drip chamber (11) comprises an outlet opening (110) being in fluid-connection with the tube (12), wherein the drip chamber (11) comprises a hydrophilic membrane (115) separating the inner volume (116) of the drip chamber (11) from the outlet opening (110).

10. Delivery arrangement, comprising an infusion set (1) according to one of the preceding claims and a covering container (4) enclosing the infusion set (1) in the delivery state.

11. Delivery arrangement according to claim 10, **characterized in that** the covering container (4) is substantially impermeable for the medical solution.
